# EUROPEAN PATENT APPLICATION

(11) **EP 2 439 260 A1**
(43) Date of publication of application: **11.04.2012**
(21) Application number: 10783103.4
(22) Date of filing: 25.05.2010
(51) Int. Cl.: C11B 9/00, A61K 8/00, A61Q 13/00

(54) **METHOD FOR SELECTING SMELL**

(30) Priority: 04.06.2009 JP 2009135245; 26.03.2010 JP 2010072076
(71) Applicant: Kao Corporation, Chuo-ku Tokyo 103-8210 (JP)
(72) Inventor: SUGIYAMA, Haruko, Tokyo 131-8501 (JP); HIKICHI, Satoshi, Tokyo 131-8501 (JP); OSHIDA, Akiko, Tokyo 131-8501 (JP)
(74) Representative: Rauh, Peter
(86) International application number: PCT/JP2010/003482
(87) International publication number: WO 2010/140315

(57) **Abstract**

Disclosed is a method which includes a step (process S2) of presenting a plurality of articles respectively scented with a plurality of fragrance candidates to a plurality of subjects; a step (process S3) of asking the subjects to smell the fragrances applied to the articles; a step (process S4) of asking the subjects to answer, with respect to the individual candidates, degrees of recollection of memory relevant to the subjects themselves in the past upon smelling of the candidates, without presenting information regarding memory to be recollected; and a step (process S5) of classifying, with respect to each candidate, a plurality of degrees of recollection of memory obtained from the plurality of subjects into a group representing larger values of the degree of recollection of memory, and a group representing smaller values of the same, and selecting a fragrance to be applied to the product, based on one, or two or more, of scores of evaluation selected from (A), (B) and (C) below used as index(es).
(A) ratio of the number of subjects, and the number of subjects in the group representing larger values of the degree of recollection of memory, for each candidate;
(B) ratio of the sum of the degrees of recollection given by the subjects in the group representing larger values of the degree of recollection, and the sum of the degrees of recollection given by the subjects in the group representing smaller values of the degree of recollection, for each candidate; and
(C) ratio of the sum of the degrees of recollection given by the subjects in the group representing larger values of the degree of recollection, and the sum of the degrees of recollection given by the subjects, for each candidate.

## Description

### TECHNICAL FIELD

The present invention relates to a method of selecting a fragrance.

### BACKGROUND ART

It has been well known that the fragrance of products affects consumer preference. Those who provide products therefore strongly appreciate selecting a fragrance which is highly preferred by consumers. There have been proposed methods of selecting highly preferred fragrances, and methods of evaluating fragrances for evaluating highly preferred fragrances. For example, there has been proposed a method of selecting a highly preferred fragrance, by using, a plural number of times, a fragrance or a product scented with a fragrance (Patent Document 1). There has been proposed another method of correctly evaluating a fragrance, by classifying terms for expressing the impression of the fragrance into sense-descriptive adjectives and mood-descriptive adjectives (Patent Document 2). Still another method reported is a method of applying fragrances together with visual and auditory stimulations, and evaluating the results of memory for the fragrance and the stimulation combinations (Patent Document 3).
Still alternatively, Patent Document 4 discloses a method in which the subjects are asked to taste foods, and to compare phrases or images shown on a concept board with their flavor-related experiences, and thereby words, emotion, thought and so forth inspired from the flavor (taste and aroma) are evaluated, so as to identify a flavor driver to be added to the food product.

### RELATED DOCUMENT

### [PATENT DOCUMENT]

[Patent Document 1] Japanese Patent Publication No. JP-A-2007-63251
[Patent Document 2] Japanese Patent Publication No. JP-A-2001-174450
[Patent Document 3] Published Japanese Translation of PCT International Application for Patent Application No. 2001-501611 [Patent Document 4] Published Japanese Translation of PCT International Application for Patent Application No. 2007-530053

### DISCLOSURE OF THE INVENTION

### [Problems to be solved by the Invention]

While Patent Documents 1 to 3 disclose methods of selecting fragrances, they only evaluate preferences and correlation with target articles, and provide no description on the evaluation of product performance.
According to the method disclosed in Patent Document 4, inspiration of the subjects may be affected by the presented concept board, since the subjects are asked to compare their flavor-related experiences with the phrases and images shown on the concept board, and to submit memory recollected by the flavor. Moreover, Patent Document 4 provides no description on the degree of recollection of memory relevant to the subjects themselves induced by the fragrance which can affect evaluation of product performance.

### [Means for Solving the Problems]

The present inventors conducted extensive investigations, based on our idea that it is necessary to select a fragrance by which the product may be highly appreciated. We consequently have found that the degree of recollection of memory relevant to the subjects themselves induced by smelling of the fragrances affected evaluation of product performance.
The present inventors also have found that a fragrance, by which the product may highly be appreciated, may be selected by calculating specific scores of evaluation of the fragrances based on the degrees of recollection of memory, and by selecting the fragrance based on the scores of evaluation.

The present invention is based on the findings described in the above.
According to the present invention, there is provided a method of selecting a fragrance to be applied to a product from a plurality of candidates. The method includes:
presenting a plurality of products respectively scented with a plurality of fragrances to a plurality of subjects;
asking the subjects to answer, with respect to each individual candidate, the degree of recollection of memory which are relevant to the subjects themselves in the past and recollected by smelling of the candidates, without presenting information regarding memory to be recollected;
classifying, with respect to each candidate, a plurality of degrees of recollection of memory obtained from the plurality of subjects into a group representing larger values of the degree of recollection of memory, and a group representing smaller values of the same; and
selecting a fragrance to be applied to the product, based on one, or two or more, scores of evaluation selected from (A), (B) and (C) below used as index(es):
   (A) ratio of the number of all subjects, and the number of subjects in the group representing larger values of the degree of recollection of memory, for each candidate;
   (B) ratio of the sum of the degrees of recollection given by the subjects in the group representing larger values of the degree of recollection, and the sum of the degrees of recollection given by the subjects in the group representing smaller values of the degree of recollection, for each candidate; and
   (C) ratio of the sum of the degrees of recollection given by the all subjects in the group representing larger values of the degree of recollection, and the sum of the degrees of recollection given by the subjects, for each candidate.

### [Effect of the Invention]

According to the present invention, a method of selecting a fragrance, capable of selecting a fragrance by which product performance of the target article may more highly be appreciated, may be provided.

### BRIEF DESCRIPTION OF THE DRAWINGS

FIG. 1 is a drawing illustrating a flow chart according to one embodiment of the present invention;
FIG. 2 is a drawing illustrating average values of each score of the evaluation of pleasantness of fragrances A to D;
FIG. 3(a) is a drawing illustrating population ratios of a group representing higher scores of evaluation of degrees of recollection of memory given by the subjects relevant to the subjects themselves in the past, with respect to each fragrances A to D, and FIG. 3(b) is a drawing illustrating ratios of sum of evaluation of the group representing higher scores of evaluation of degrees of recollection of memory given by the subjects relevant to the subjects themselves in the past;
FIG. 4 is a drawing illustrating scores of evaluation of product performances given by the groups representing higher and lower scores of evaluation of emotional intensity of memory given by the subjects relevant to the subjects themselves in the past, with respect to all fragrances A to D;
FIG. 5 is a drawing illustrating scores of evaluation of product performances given by the groups representing higher and lower scores of evaluation of emotional intensity of memory given by the subjects relevant to the subjects themselves in the past, with respect to the fragrance A;
FIG. 6 is a drawing illustrating fragrance-wise scores of evaluation of product performances, given by the group representing higher scores of evaluation of emotional intensity of memory given by the subjects relevant to the subjects themselves in the past, with respect to all fragrances A to D;
FIG. 7 is a drawing illustrating average values of scores of evaluation of pleasantness of fragrances X to Z;
FIG. 8(a) is a drawing illustrating population ratios of a group representing higher scores of evaluation of degrees of recollection of memory given by the subjects relevant to the subjects themselves in the past, with respect to fragrances X to Z, and FIG. 8(b) is a drawing illustrating ratios of the sum of evaluation of the group representing higher scores of evaluation of the degrees of recollection of memory given by the subjects relevant to the subjects themselves in the past;
FIG. 9 is a drawing illustrating scores of evaluation of product performances given by the groups representing higher and lower scores of evaluation of emotional intensity of memory given by the subjects relevant to the subjects themselves in the past, with respect to fragrances X to Z; and
FIG. 10 is a drawing illustrating scores of evaluation of product performances given by the groups representing higher and lower scores of evaluation of emotional intensity of memory given by the subjects relevant to the subjects themselves in the past, with respect to fragrance X.

### DESCRIPTION OF THE EMBODIMENTS

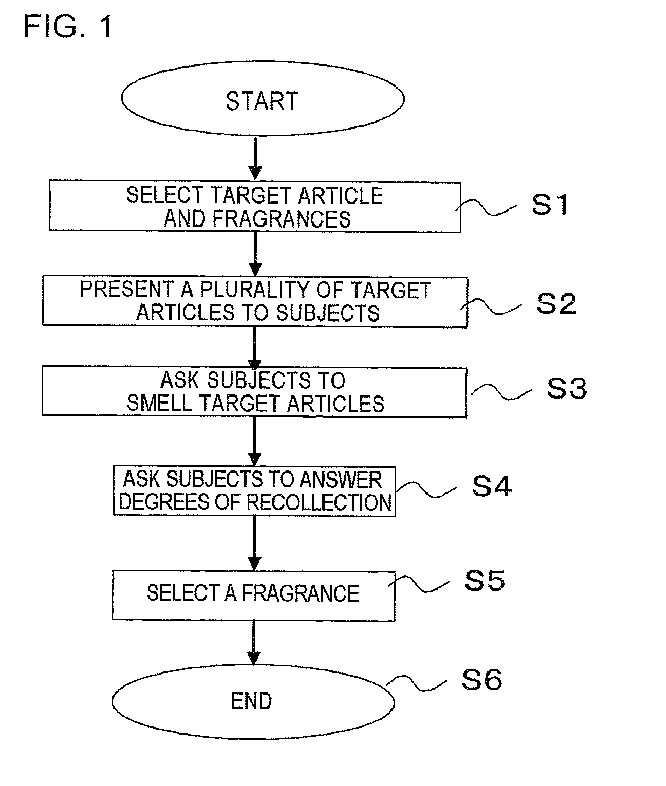

Embodiments of the present invention will be explained below, referring to the attached drawings.
First, an outline of the method of selecting a fragrance of this embodiment will be explained, referring to FIG. 1.
The method of selecting a fragrance of this embodiment is a method of selecting a fragrance to be applied to a product, from a plurality of candidates of fragrances to be applied to a product. The method includes a step (process S2) of presenting a plurality of articles (products) respectively scented with a plurality of candidates to subjects; a step (process S3) of asking the subjects to smell the fragrances applied to the articles; a step (process S4) of asking the subjects to answer, with respect to each candidate, degrees of recollection of memory which are recollected by smelling of the candidates and relevant to the subjects themselves in the past, without presenting information regarding memory to be recollected; and a step (process S5) of selecting, from the plurality of candidates, a fragrance to be applied to the product, based on the degree of recollection of memory used as an index.

Next, the method of selecting a fragrance of this embodiment will be detailed below.

### (Process S1)

In advance of executing Process S2 to Process S5, this process is provided for executing a procedure of presenting a fragrance group, consisting of a plurality of fragrances, to the subjects; a procedure of obtaining answers from the subjects about preference of each fragrance contained in the fragrance group; and, a procedure of selecting a partial plurality of fragrances from the fragrance group, in decreasing order of preference including the highest preference, so as to select fragrance candidates to be applied to the product (or may simply be referred to as "candidates,", hereinafter).
First, in this process, a target article to be scented with a fragrance is set, and the above-described fragrance group is set. While the target article to be scented with the fragrance is not specifically limited, it includes not only personal-use products including hair cleansing agents such as shampoo and rinsing agent, cosmetics such as lotion and milky lotion, foundation and makeup materials, but also household products such as house-keeping cleaning agent and laundry detergent. The method of selecting a fragrance of the present invention is more effective when applied to personal-use products in which personal preference is strongly reflected.

Each fragrance in the fragrance group to be presented may be composed of a single fragrant-component or may be a fragrant compound containing a plurality of fragrant components mixed therein.
Each fragrance in the fragrance group is set depending on attributes of the main target demographic (age, sex, cultural background, and so forth) of the article to which the fragrance is applied. It is preferable to evaluate the preference of the subjects, for each of the thus-set fragrances in the fragrance group (for example, for 10 to 20 species of fragrances). The fragrances are then classified into a group of fragrances given a high preference and a group of fragrances given a low preference, and the plurality of fragrances which belong to the group representing a high preference are selected as candidates to be applied to the target article. For example, the candidates may be selected from the top-two to top-eight fragrances in the thus-set fragrance group, ranked by high average scores of the evaluation preference given by the subjects, or may be selected from the top-two to top-eight fragrances ranked by the number of subjects or ratio of subjects who gave high scores of preference.

The evaluation of the preference of fragrance may contain one or more items relevant to a good feeling or emotion induced by the fragrance.
The items for evaluation of fragrance typically include a good feeling or emotion, such as positive, joyful, relaxing, calm, fresh, revitalizing, energetic, warm, safe, satisfactory, happy, and so forth.
Scale of evaluation is given by numerical conversion of a range between a point which represents that no feeling or emotion listed in the items of evaluation was induced upon smelling of the fragrance, and a point which represents that a strong feeling or emotion listed in the items of evaluation was induced. The scale of evaluation is preferably a so-called linear visual analog scale, and more preferably a scale using scores, which is exemplified as an ordinal scale with associated scores, an interval scale with associated scores, and a ratio scale with associated scores. For an exemplary case where the preference of fragrances is evaluated by nine grades (grade 1: very unpleasant, up to grade 9: very pleasant), the scores of evaluation which represent a high preference may include grade 6 or above, or include grades 7 to 9.
By the evaluation of preference as described above, the candidates to be applied to the target article are selected. While the selection of candidates is not always necessary, another possible method of selection may be such as selecting fragrances frequently used for the target article and those frequently used in similar fields of the target article; or such as selecting them based on a preference of fragrances and evaluation of feeling (neat impression, stability) required for products in the related product field of the target article.

### (Process S2 to Process S4)

In this process, each of the plurality of candidates selected in process S1 is applied to the target article, and presented to the subjects. The subjects smell each the fragrance applied to the target article. Subjects are then asked to answer, with respect to each candidate, degrees of recollection of memory that are recollected by smelling of the candidates and relevant to the subjects themselves in the past, without being presented with information regarding memory to be recollected.
The subjects preferably represent attributes of the main target demographic of the article. Each subject may smell all fragrances applied to the target article, or each of a plurality of the subjects may smell different fragrance applied to the target article.
The number of subjects is preferably two or more, and typically eight or more, and more preferably ten or more, from the viewpoint of ensuring accuracy of the degree of recollection. While a larger number of subjects is more preferable, it may be 70 or smaller, or may be 50 or smaller.

When the subjects are allowed to recollect memory relevant to the subject themselves in the past, they are allowed to freely recollect it, without being directed (specified) with information regarding memory to be recollected.
Note that it is permissible to submit a visible or audible question, in order to help recollection of the memory relevant to the subjects themselves in the past.
For example, a direct question such as "Did this fragrance make you recollect some memory?" may be submitted through their visual sense or auditory sense, or other types of questions such as "Think about what you recollect, after you smelled the fragrance of this target article", or "Feel your own experience, when you smell the fragrance of this target article".
In this process, the subjects are allowed to freely recollect their memory, without being given any direction which directly teaches memory to be recollected, nor any indirect direction which presents something relevant to the memory to be recollected other than the fragrance. More specifically, any question such as specifying the memory to be recollected (such as a question of asking feel of use of cosmetics that the subjects had ever used in the past) is not submitted, and any photograph, picture, music and so forth same as, or equivalent to, stimulus ever experienced by the subjects in the past, for the purpose of indirectly identifying details of memory to be recollected, are not given. By avoiding direct or indirect direction or identification of details of memory to be recollected to the subjects, any factors possibly prevent the subjects from recollecting their memory relevant to themselves in the past may be eliminated, and makes them possible to naturally recollect their memory relevant to themselves in the past inspired by the fragrance.

It is to be understood now that the memory relevant to the subjects themselves in the past includes so-called autobiographical memory. "The past" is not provided for the purpose of selection of fragrance, but means the past background of each subject. The fragrance and the memory relevant to the subjects themselves in the past are linked to each other on an instinctive level, so that the subjects recollect the memory relevant to the subjects themselves in the past, induced upon smelling of the fragrance. The level of intensity of recollection is evaluated in terms of degree of recollection of memory (simply referred to as "degree of recollection", hereinafter).
The memory relevant to the subjects themselves in the past include, for example, "a tour around Hawaii where I stayed in my teenage years", "memory of my grandmother whom I visit every summer vacation", "impression of a deodorant spray which I used for the first time", and so forth.
The subjects answer the degree of recollection of memory relevant to the subjects themselves in the past.
The degree of recollection is obtainable by evaluating at least one item selected, for example, from intensity of emotion recollected by memory, intensity of sense of being brought back to the past, clearness of memory, whether the scene of memory comes to my mind, feeling of just like the memory is re-experienced, whether the fragrance may be felt as something special, whether the fragrance may be felt as something important, whether the place, object and person relevant to the memory may additionally be recollected, and degree of detail of the memory.
Among them, at least one of intensity of emotion recollected by memory, intensity of sense of being brought back to the past, and clearness of memory is preferably evaluated, and in particular, intensity of emotion recollected by memory, and intensity of emotion of sense of being brought back to the past are preferably evaluated.
By evaluating the degree of recollection based on a plurality of items as described in the above, accuracy of the degree of recollection may be improved. Since intensity of emotion recollected by memory, intensity of sense of being brought back to the past, and clearness of memory are highly correlated items, the accuracy of the degree of recollection may be ensured by evaluating these items.
The degree of recollection is preferably expressed by a multi-grade evaluation, and is therefore evaluated in this embodiment by converting the results into scores.
For example, the degree of recollection relevant to the intensity of emotion recollected by memory is converted into a score over a range between a point which represents very weak emotional intensity, and a point which represents very strong emotional intensity.
On the other hand, the degree of recollection relevant to the intensity of sense of being brought back to the past, induced upon smelling of the fragrance, is converted into a score over a range between a point which represents absolutely no sense of being brought back, and a point which represents a perfect sense of being brought back to the past.
The degree of recollection relevant to clearness of memory, induced upon smelling of the fragrance, is converted into a score over a range between a point which represents that details of the memory are ambiguous as a whole, and a point which represents that they are very clear.
While the subjects in the above were asked to answer the degrees of recollection of memory relevant to the subjects themselves in the past, the subjects may alternatively be asked to answer also memory relevant to the subjects themselves in the past, followed by an answer of the degrees of recollection.

### (Process S5)

In this process, a fragrance to be applied to the target article is selected, based on the degrees of recollection obtained from the subjects.
First, with respect to each candidate, a plurality of degrees of recollection of memory obtained from the plurality of subjects are classified into a group representing larger values of the degree of recollection of memory, and a group representing smaller values of the same.
For example, since the degrees of recollection are evaluated by multi-grade scores, with respect to each candidate, the degrees of recollection obtained from the plurality of subjects are classified into the group representing larger values and the group representing smaller values, using the median value of the numerical range of the multi-grade evaluation as a reference.
Alternatively, the top 20 to 50% of the numerical range of the multi-grade evaluation may be classified into the group representing larger values, and the residual portion may be classified into the group representing smaller values.
Then, the fragrance is selected based on one, or two or more, of scores of evaluation selected from (A), (B) and (C) below used as index(es):
(A) ratio of the number of subjects, and the number of subjects in the group representing larger values of the degree of recollection of memory, for each candidate;
(B) ratio of the sum of the degrees of recollection given by the subjects in the group representing larger values of the degree of recollection, and the sum of the degrees of recollection given by the subjects in the group representing smaller values of the degree of recollection, for each candidate; and
(C) ratio of the sum of the degrees of recollection given by the subjects in the group representing larger values of the degree of recollection, and the sum of the degrees of recollection given by the subjects, for each candidate.
Any one of (A), (B) and (C) may be used as the index, or a plurality of scores of evaluation selected from (A), (B) and (C) may be used as the indexes.

The method of selecting a fragrance using (A) as the index will be explained.
A plurality of degrees of recollection obtained from the plurality of subjects, for each candidate, is classified into the group representing larger values, and the group representing smaller values. Thereafter, for each candidate, the ratio of the number of subjects and the number of subjects in the group representing larger values of the degree of recollection of memory, which is exemplified by the ratio of the number of subjects in the group representing larger values of the degree of recollection of memory, with respect to the number of subjects, for each candidate, is calculated. Then, for example, fragrance(s) given the highest ratio of the number of subjects in the group representing larger values of the degree of recollection of memory is selected. The number of fragrance(s) selected herein is not always necessarily one, but may be two or more. For example, a partial plurality of fragrances may be selected from the candidates in decreasing order of population ratio of the subjects in the group representing larger values of the degree of recollection of memory including the largest population ratio.
For the case where a plurality of items for evaluating the degree of recollection are adopted (for example, for the case where the degree of recollection is evaluated for each of three items of intensity of emotion recollected by memory (item 1), intensity of sense of being brought back to the past (item 2), and clearness of memory (item 3)), the fragrance is selected typically as described below.
For each item, the plurality of degrees of recollection obtained from the plurality of subjects are classified into the group representing larger values and into the group representing smaller values, and population ratio of the number of subjects in the group representing larger values, relative to the number of the subjects is calculated for each candidate. Then, for each candidate, population ratios of the group representing larger values for the individual items (population ratio of the group representing larger values for item 1 + population ratio of the group representing larger values for item 2 + population ratio of the group representing larger values for item 3) are summed up, and a fragrance given the largest value is selected.
Alternatively, the plurality of degrees of recollection obtained from the plurality of subjects is classified into the group representing larger values, and into the group representing smaller values. Then, the number of subjects in the group representing larger values for the individual items may be summed (the number of subjects in the group representing larger values for item 1 + the number of subjects in the group representing larger values for item 2 + the number of subjects in the group representing larger values for item 3), and a fragrance given the largest value of ratio relative to the number of all subjects may be selected.

Next, the method of selecting a fragrance using (B) as the index will be explained.
For each candidate, the plurality of degrees of recollection obtained from the plurality of subjects is classified into the group representing larger values, and into the group representing smaller values. Next, for each candidate, the sum of the degrees of recollection given by the subjects in the group representing larger values, and the sum of the degrees of recollection in the group representing smaller values for each candidate, is calculated.
Next, for each candidate, the ratio of the sum of the degrees of recollection given by the subjects in the group representing larger values of the degree of recollection, and the sum of the degrees of recollection given by the subjects in the group representing smaller values of the degree of recollection, is calculated. For example, the ratio expressed by (the sum of the degrees of recollection given by the subjects in the group representing larger values of the degree of recollection/the sum of the degrees of recollection in the group representing smaller values of degree of recollection) is calculated. Then, fragrance(s) given the highest ratio is selected. The number of fragrance(s) selected herein is not always necessarily one, but may be two or more. For example, a partial plurality of fragrances may be selected from the plurality of candidates, in decreasing order of the ratio of the sum of the degrees of recollection given by the subjects in the group representing larger values of the degree of recollection of memory, relative to the sum of the degrees of recollection given by the subjects in the group representing smaller values of the degree of recollection of memory (the sum of the degrees of recollection in the group representing higher degrees of recollection/the sum of the degrees of recollection in the group representing lower degrees of recollection), including the highest one.
For the case where a plurality of items for evaluating the degree of recollection are adopted (for example, for the case where the degree of recollection is evaluated for each of three items of intensity of emotion recollected by memory (item 1), intensity of sense of being brought back to the past (item 2), and clearness of memory (item 3)), the sum of the degrees of recollection of the group representing larger values, and the sum of the degrees of recollection of the group representing smaller values, are calculated for each item.
Then, for example, a grand total of the sums of the degrees of recollection of the group representing larger values for the individual items (the sum of the degrees of recollection of the group representing larger values for item 1 + the sum of the degrees of recollection of the group representing larger values for item 2 + the sum of the degrees of recollection of the group representing larger values for item 3), and a grand total of the sums of the degrees of recollection of the group representing smaller values of the degree of recollection for the individual items (the sum of the degrees of recollection of the group representing smaller values for item 1 + the sum of the degrees of recollection of the group representing smaller values for item 2 + the sum of the degrees of recollection of the group representing smaller values for item 3), are calculated for each candidate, and a fragrance given the largest value of the ratio expressed by (grand total of the sums of the degrees of recollection of the group representing larger values of the degree of recollection/grand total of the sums of the degrees of recollection of the group representing smaller values of the degree of recollection) is selected.

The method of selecting a fragrance using (C) as the index will further be explained.
The plurality of degrees of recollection of memory obtained from the plurality of subjects for each candidate are classified into the group representing larger values, and into the group representing smaller values. Next, the ratio of the sum of the degrees of recollection given by the subjects in the group representing larger values of the degree of recollection and the sum of the degrees of recollection given by the subjects, which is exemplified by the ratio of the sum of the degrees of recollection given by the subjects in the group representing larger values of the degree of recollection of memory, relative to the sum of the degrees of recollection given by the subjects, is calculated.
Then, for example, a fragrance given the largest ratio of the sum of the degrees of recollection given by the subjects in the group representing larger values of the degree of recollection of memory, relative to the sum of the degrees of recollection given by all subjects (the sum of the degrees of recollection given by the subjects in the group representing larger values of the degree of recollection of memory/the sum of the degrees of recollection given by the subjects), is selected. The number of fragrances selected herein is not always necessarily one, but may be two or more. For example, a partial plurality of fragrances may be selected from the plurality of candidates, in decreasing order of the ratio expressed by (the sum of the degrees of recollection given by the subjects in the group representing larger values of the degree of recollection of memory/the sum of the degrees of recollection given by the subjects), or a partial plurality of fragrances may be selected in decreasing order of the ratio.
For the case where a plurality of items for evaluating the degrees of recollection are adopted (for example, for the case where the degrees of recollection is evaluated for each of three items of intensity of emotion recollected by memory (item 1), intensity of sense of being brought back to the past (item 2), and clearness of memory (item 3)), the sum of the degrees of recollection given by the subjects in the group representing larger values, and the sum of the degrees of recollection given by all subjects, are calculated for each item.
Then, for each candidate, for example, a grand total of the sums of the degrees of recollection given by the subjects in the group representing larger values for the individual items (sum for item 1 + sum for item 2 + sum for item 3), and a grand total of the sums of the degrees of recollection given by all subjects for all items, are calculated, and a fragrance given the largest value of the ratio of the grand total of the sums of the degrees of recollection of the group representing larger values, relative to the grand total of the sums of the degrees of recollection given by all subjects, is selected.

The fragrance selected as described in the above is defined as the fragrance to be applied to the target article.
By the selection as described in the above, based on the degrees of recollection of memory relevant to the subjects themselves in the past, a fragrance by which product performances of the target article may more highly be appreciated, is now selectable.
In addition, a fragrance by which product performances of the target article may more highly be appreciated is now selectable in an appropriate manner, by classifying, for each candidate, the plurality of degrees of recollection of memory obtained from the plurality of subjects into the group representing larger values of the degree of recollection of memory, and into the group representing smaller values, and by calculating the scores of evaluation of each candidate based on (A) the population ratio of the group representing larger degrees of recollection of memory; (B) the ratio of the sum of the degrees of recollection of the group representing larger degrees of recollection, and the sum of the degrees of recollection of the group representing smaller degrees of recollection; or (C) the ratio of the sum of the degrees of recollection given by the subjects in the group representing larger values of the degree of recollection, relative to the sum of the degrees of recollection given by all subjects. In other words, by scenting the product with the fragrance given a larger population ratio of the group representing larger values of the degree of recollection of memory, a larger number of consumers will highly appreciate performances of the products. In addition, by scenting the product with the fragrance given the ratio of the sum of the degrees of recollection of the group representing larger values of the degree of recollection of memory, relative to the sum of the degrees of recollection of the group representing lower degrees of recollection of memory, or to the sum of the values of degree of recollection of memory given by all subjects, it is expected to have the consumers who highly appreciate performances of the products.
Note that the above-described process S1 to process S5 precedes use of the products by the subjects.
In this way, the degree of recollection of memory induced by the fragrance per se may correctly be evaluated, without being affected by evaluation of functionality inherent to the product.

### [Examples]

### (Example 1)

Next, Example 1 of the present invention will be explained below.

### (Process S1)

First, a target article to be scented with a fragrance was determined, and the candidates for the fragrance to be applied to the target article were selected. In this Example, a body lotion was used as the target article. Next, 17 kinds of fragrances were selected to configure a fragrance group. Taking properties of a main target demographic of the body lotion into consideration, 54 American women aged 22 to 31, and 68 American women aged 42 to 51, for a total of 122, were recruited as the subjects.

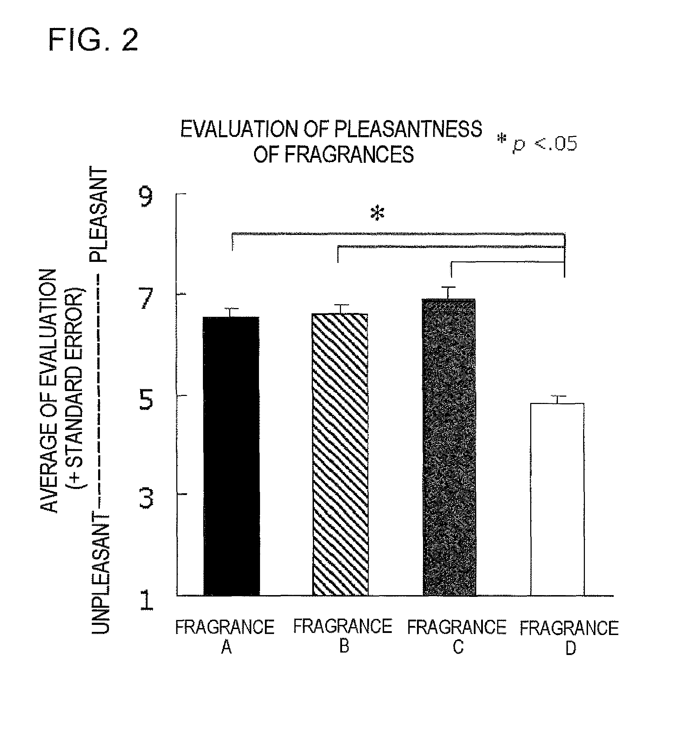

First, each of 17 kinds of fragrances was applied to the body lotion of the same composition, and the preference of 122 subjects was evaluated. The preference was evaluated by asking the subjects to select a single item from a nine-grade criteria asking "How much do you like this fragrance?", ranging from "1: do not like very much" to "9: like very much".
The obtained answer data regarding evaluation of preference given by the subjects were compiled for each fragrance, and average values were calculated. The seventeen kinds of fragrances were classified based on the average values of the collected data of preference, into a high-preference group and a low-preference group, and three kinds of fragrances A to C, which are the top three of the preference in the high-preference group, and an additional fragrance D in the high-preference group, totaled four kinds of fragrances, were selected as the candidates.
The thus-selected four kinds of candidates are shown in Table 1. The fragrance A has an aroma of vanilla categorized into Oriental note, the fragrance B has an aroma of grapefruit categorized into citrus note, the fragrance C is categorized into fruity note, and the fragrance D has a cassis-based aroma categorized into green-floral note. FIG. 2 illustrates average values of scores of evaluation of pleasantness about each fragrance of A to D, evaluated in this process. No distinctive differences were found among the fragrances A to C, whereas a difference was found between a group of the fragrances A to C and fragrance D.

**[Table 1]**

| | Note |
|---|---|
| Fragrance A | Oriental note |
| Fragrance B | Citrus note |
| Fragrance C | Fruity note |
| Fragrance D | Green-Floral note |

### (Processes S2 to S4)

Next, the four candidates listed in Table 1 were respectively applied to body lotions. All body lotions herein have the same composition. The body lotions were presented to the subjects, and the subjects were asked to smell the fragrances (of the lotions). The subjects recruited herein were American women aged from 22 to 31, among which 71 women smelled the body lotion scented with the fragrance A (perfume A), 67 women smelled the body lotion scented with the fragrance B (perfume B), 68 women smelled the body lotion scented with the fragrance C (perfume C), and 65 women smelled the body lotion scented with the fragrance D (perfume D).

The subjects were asked to smell the fragrance of the individual body lotions. After that, they were allowed to recollect memory relevant to the subjects themselves in the past for each candidate, and to answer the degrees of recollection of memory.
No information regarding memory to be recollected was directed to the subjects during the process of allowing the subjects to recollect memory relevant to the subjects themselves in the past. Instead, announcements for assisting recollection of memory relevant to the subjects themselves were given. The announcements for assisting recollection of memory were such as those asking "Some memory arose from the fragrance?" and "What kind of memory was it?". Specific examples are shown below.
"Let's smell the fragrance. While you are smelling the fragrance, think that the fragrance brings you a certain kind of mood, and that the fragrance is somehow meaningful to you. Did this fragrance make you recollect some memory? What kind of memory is it? Think about the fragrance for a while. You need not name the fragrance, or may be associated with something or nothing by the fragrance. We are interested in what you are thinking and what you are feeling during your experience with the fragrance. There is no right answer or wrong answer. Just give us your honest comments."
Next, the subjects were asked to freely describe details of the memory recollected (memory regarding the subjects themselves in the past).

The subjects were then asked to answer the degrees of recollection of memory relevant to the subjects themselves in the past. The items for evaluating the degree of recollection were those relevant to the state of recollection (property of memory) and intensity thereof. More specifically, the questions herein included three items with respect to the recollected memory induced upon smelling of the fragrance, such as, how much the emotional intensity was (emotional intensity of memory, evaluation item 1), how strongly did you feel the sense of being brought back to the time and place your memory arose (sense of being brought back, evaluation item 2), and how clear or how ambiguous was the memory (clearness of memory, evaluation item 3).
With respect to the emotional intensity of memory induced upon smelling of the fragrance, the subjects were asked to answer by selecting a number from a nine-grade criteria ranging from "1: very weak" to "9: very strong". With respect to the degree of sense being brought back to the time when the autobiographical memory arose, the subjects were asked to answer by selecting a number from a nine-grade criteria ranging from "1: not brought back at all" to "9: perfectly brought back". With respect to the clearness of memory, the subjects were asked to answer by selecting a number from a nine-grade criteria ranging from "1: ambiguous correlation as a whole" to "9: very clear even".

### (Process S5)

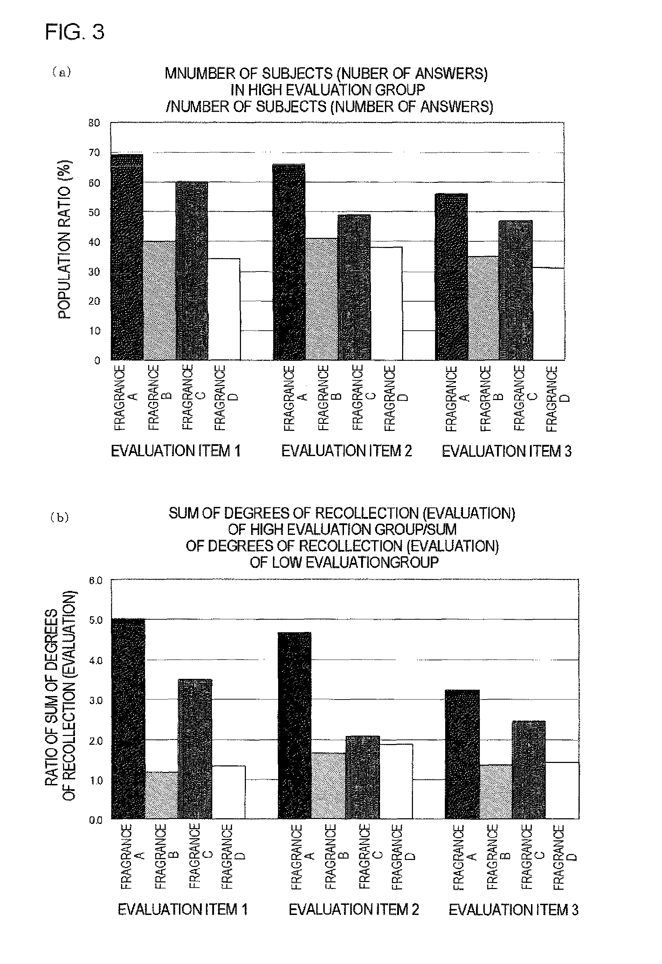

Next, the answer data regarding the degrees of recollection given by the subjects obtained in the previous process were compiled, and a fragrance to be applied to the target article was selected.
Since the degrees of recollection were obtained in the form of multi-grade evaluation with scores 1 to 9 for the individual evaluation items, so that the number of subjects was classified, for each item, into the those yielding low answer data from grade 1 to grade 5 (or, the group representing smaller values of the degree of recollection, occasionally referred to as low evaluation group, hereinafter), and into those yielding high answer data from grade 6 to grade 9 (or, the group representing larger values of the degree of recollection, occasionally referred to as high evaluation group, hereinafter). Also, for the individual candidates, the number of subjects yielding the answer data in the high evaluation group was counted, and the ratio thereof relative to the number of all subjects was calculated. Also, sum of the answer data of the high evaluation group, sum of the answer data of the low evaluation group, sum of the scores of evaluation given by all subjects, and, ratio of the sum of the answer data of the high evaluation group relative to the answer data of the low evaluation group, and ratio of the sum of the answer data of the high evaluation group relative to the sum of the scores of evaluation given by all subjects, were calculated.
Table 2 shows, for each of the evaluation items of the degrees of recollection of the fragrances A to D, the number of answers (the number of subjects) in the high evaluation group and population ratio thereof relative to the number of all subjects (values in the parentheses in Table 2), and, sum of the answer data of the high evaluation group, ratio of the sum of the answer data of the high evaluation group relative to the sum of the answer data of the low evaluation group, and ratio of the sum of the answer data of the high evaluation group relative to the sum of the answer data. On the other hand, FIG. 3(a) illustrates ratios of the number of answers of the high evaluation group relative to the number of all subjects, with respect to the evaluation items of the degree of recollection for each fragrance A to D, and FIG. 3(b) illustrates ratios of sum of the answer data of the high evaluation group relative to the sum of the answer data of the low evaluation group (sum of the answer data of the high evaluation group/sum of the answer data of the low evaluation group), with respect to the evaluation items of the degree of recollection for the fragrances A to D.

**[Table 2]**

| | Number of subjects | Evaluation item 1 | | | | Evaluation item 2 | | | | Evaluation item 3 | | | |
|---|---|---|---|---|---|---|---|---|---|---|---|---|---|
| | | High evaluation group | | | | High evaluation group | | | | High evaluation group | | | |
| | | Number of answers (population ratio) | Sum of degrees of recollection (*1) | Ratio (*2) | Ratio (*3) | Number of answers (population ratio) | Sum of degrees of recollection (*1) | Ratio (*2) | Ratio (*3) | Number of answers (populatio n ratio) | Sum of degrees of recollection (*1) | Ratio (*2) | Ratio (*3) |
| Fragrance A | 71 | 49(69%) | 350 | 5.0 | 83% | 47(66%) | 350 | 4.7 | 82% | 40(56%) | 298 | 3.2 | 76% |
| Fragrance B | 67 | 27(40%) | 183 | 1.2 | 54% | 28(42%) | 205 | 1.6 | 62% | 24(36%) | 179 | 1.3 | 57% |
| Fragrance C | 68 | 41(60%) | 297 | 3.5 | 78% | 33(49%) | 246 | 2.1 | 68% | 32(47%) | 242 | 2.5 | 71% |
| Fragrance D | 65 | 22(34%) | 160 | 1.3 | 57% | 25(38%) | 179 | 1.9 | 65% | 20(31%) | 151 | 1.4 | 59% |

| | | | | | | | | | | | | | |
|---|---|---|---|---|---|---|---|---|---|---|---|---|---|
| Item 1: Evaluation of emotional intensity of memory given by the subjects relevant to the subjects themselves in the past. Item 2: Evaluation of intensity of sense of being brought back to the time when memory given by the subjects relevant to the subjects themselves in the past arose. Item 3: Evaluation of clearness of memory given by the subjects relevant to the subjects themselves in the past. Number of answers: the number of answers by the high evaluation group (the number of subjects in the high evaluation group), Copulation ratio=(the number of answers by the high evaluation group)/(the number of all subjects) (*1): Sum of the degrees of recollection given by the subjects in the high evaluation group (*2): (Sum of the degrees of recollection given by the subjects in the high evaluation group)/(sum of the degrees of recollection given by the subjects in the low evaluation group) (*3): (Sum of the degrees of recollection given by the subjects in the high evaluation group)/(sum of the degrees of recollection given by the subjects) | | | | | | | | | | | | | |

As shown in Table 2, fragrance A and fragrance C were given a large number of subjects in the group representing higher degrees of recollection, with respect to the emotional intensity of memory given by the subjects relevant to the subjects themselves in the past (evaluation item 1), and also given large values of {sum of the degrees of recollection (answer data) of the high evaluation group/sum of the degrees of recollection (answer data) of the low evaluation group}. In particular, with respect to fragrance A, a half or more subjects were classified into the high evaluation group for all evaluation items, showing largest population ratios of the high evaluation group over the other fragrances, for all evaluation items.
Fragrance A was also found to show large values of {the sum of the degrees of recollection of the high evaluation group/the the sum of the degrees of recollection given by the low evaluation group} for all evaluation items, and was further found to show large values of the ratios of the sum of degrees of recollection ratio by the high evaluation group, relative to the sum of the degrees of recollection, for all evaluation items.
These results teach that the target article scented with fragrance A was given a large number of subjects whose degrees of recollection of memory relevant to the subject themselves in the past are high.
Fragrances A and C showed no distinctive difference in between as for the scores of evaluation of preference (pleasantness of fragrances) illustrated in FIG. 2, even showing a slight advantage of fragrance C. Fragrance A, however, showed higher scores of evaluation with respect to the evaluation items regarding the memory given by the subjects relevant to the subjects themselves in the past, and this enabled selection of fragrance A which could not be selected based on the evaluation of preference.

### (Examination)

Next, performances of the target article were evaluated. More specifically, after evaluation of the degree of recollection of memory given by the subjects relevant to the subjects themselves in the past, each subject used, for a week, the body lotion which is the target article scented with the fragrance evaluated by the subject, and thereafter preference of the target article and product performances of the target article were evaluated. The evaluation of preference of the target article after use was conducted so that the subject was instructed to "choose the most appropriate description as for the degree of likeness or unlikeness of this product", and choose one item from "1: dislike it very much", "2: dislike it slightly", "3: do not care", "4: like it slightly", and "5: like it very much".
The evaluation of the product performances of the target article was conducted so that the subjects were asked to choose from any one value from a nine-grade criteria ranging from "1: do not think so at all" to "5: do not care" to "9: think so very much", for each of 20 evaluation items of product performances listed in Table 3.

**[Table 3]**

| Product Performance | Product Performance |
|---|---|
| Evaluation Items 1 | Evaluation Items 2 |
| Protests my skin from drying. | Special to me. |
| Protects my skin from roughening. | Makes my skin look more nicely. |
| Keeps the skin soft. | Helps my better skin care. |
| Keeps the skin smooth. | Makes my skin look more healthy. |
| Smoothly spreadable. | Provides a silky touch to my skin. |
| Quickly absorbed. | Makes my skin brighter. |
| Fully absorbed. | Makes my skin firm and elastic. |
| Not oily on the skin. | Fees good by using it. |
| Long-lasting moistening care goods. | Makes my skin look younger. |
| Strongly moistens my skin. | |
| Body moistening care goods for daisy use. | |

The evaluation of product performance listed in Table 3 may be classified into a first group (product performance evaluation items 1) and a second group (product performance evaluation items 2). The first group relates to functionality of the body lotion including "smoothly spreadable", "quickly absorbed" and so forth, which can also be evaluated in an objective manner typically by physical evaluation. The second group relates to emotional performance evaluation items which are rather subjective such as "special to me", "makes my skin more look more nicely" and so forth, which are difficult to be evaluated in an objective manner typically by physical evaluation.

Data of the product performances after use of the target article was analyzed in relation to the degrees of recollection of memory relevant to the subjects themselves in the past which was rated before the target article usage. More specifically, for each evaluation item of the degrees of recollection of memory which are relevant to the subjects themselves in the past and are collected before use, an average value of the product performance evaluation data given by the subjects who belong to the high evaluation group of the degree of recollection (grades 7 to 9), and an average value of the product performance evaluation data given by the subjects who belong to the very low evaluation group of the degree of recollection (grades 1 to 3), were compared.

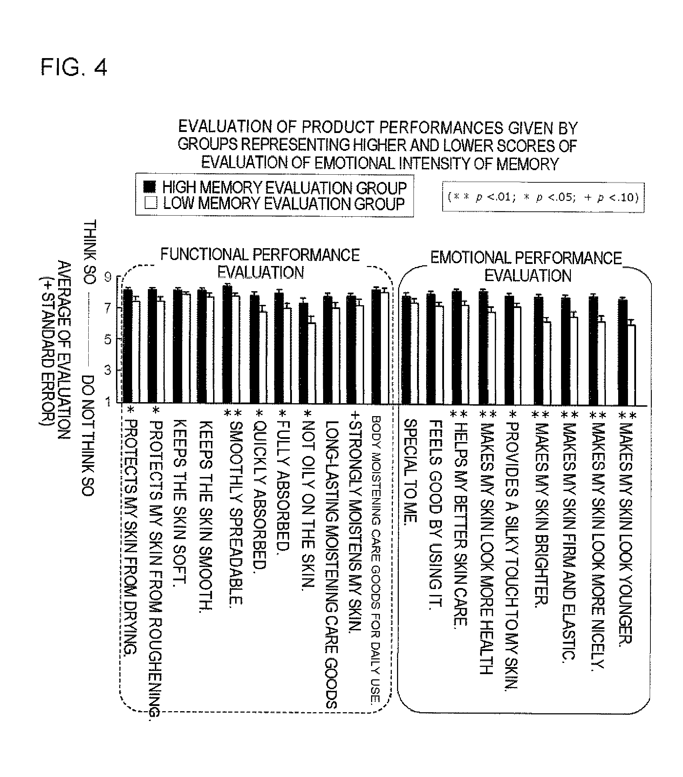

FIG. 4 illustrates the average values of the product performance evaluation given by the high evaluation group representing high scores of evaluation of the evaluation item 1, and the average values of the product performance evaluation given by the low evaluation group representing low scores of evaluation of the evaluation item 1, with respect to all of the fragrances A to D. The evaluation item 1 is the emotional intensity of the recollected memory relevant to the subjects themselves in the past which was rated before the target article usage. The evaluation item 1 is one of the evaluation items regarding the degrees of recollection of memory relevant to the subjects themselves in the past.
As illustrated in FIG. 4, the product performance evaluation given by the subjects in the group representing high degrees of recollection for the evaluation items 1, was found to be higher than the product performance evaluation given by the subjects in the group representing low degrees of recollection for that, and significant differences (p<0.01) were confirmed in six items in the emotional performance evaluation. It is considered that high product performance evaluation is obtained, when the degrees of recollection of memory given by the subjects relevant to the subjects themselves in the past and recollected before use, are high.

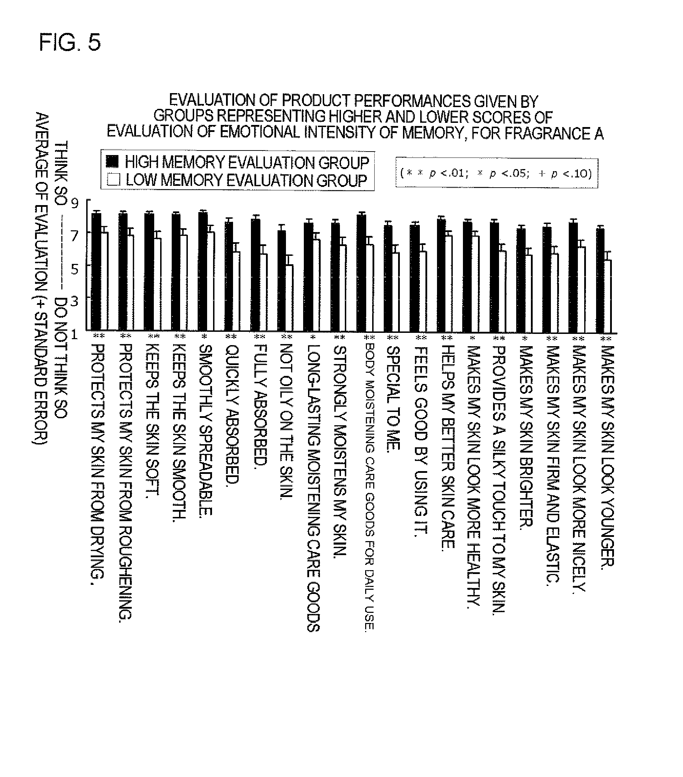

FIG. 5 illustrates average values of the product performance evaluation with respect to the fragrance A, given by the groups representing high and low degrees of recollection of memory given by the subjects relevant to the subjects themselves in the past (evaluation item 1). The group representing high degrees of recollection was found to give high scores of evaluation in almost all items of product performance evaluation, including the items of functional performance evaluation, with significant differences (p<0.01) in 16 items out of 20 items.

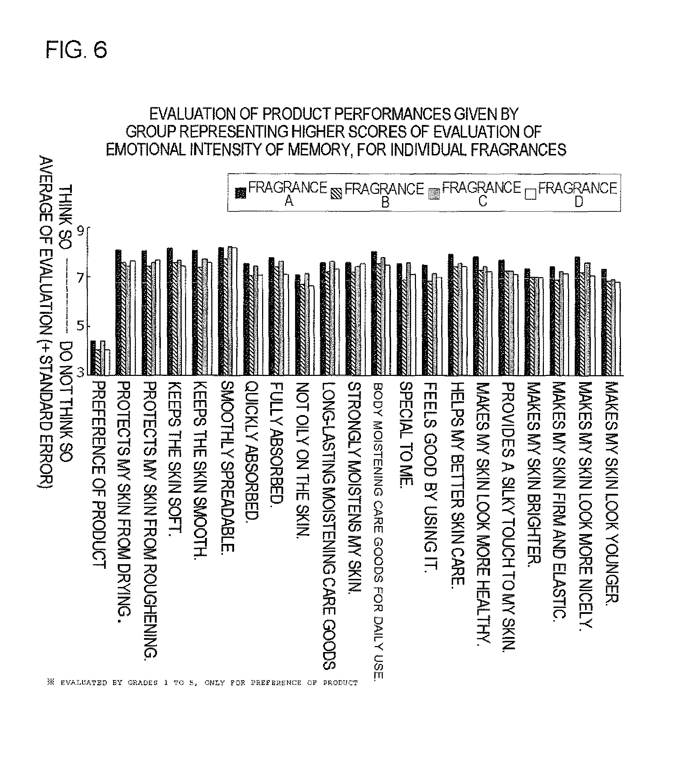

The results of comparison, for each fragrance, of average scores of the product performance evaluation with respect to the evaluation items regarding emotional intensity of memory, given by the subjects in the group representing high degrees of recollection, are illustrated in FIG. 6. As can be seen in FIG. 6, the fragrance A, given the largest population ratio of the subjects in the high evaluation group, and also given the largest ratio of sum of the answer data given by the subjects in the high evaluation group, was found to give higher average scores of the product performance evaluation, as compared with the other fragrances.

### (Example 2)

Next, Example 2 of the present invention will be explained. A body lotion was used as the target article, and 43 to 49 American women aged from 22 to 31 were recruited as the subjects, taking properties of people who are targeted to buy the body lotion into consideration.

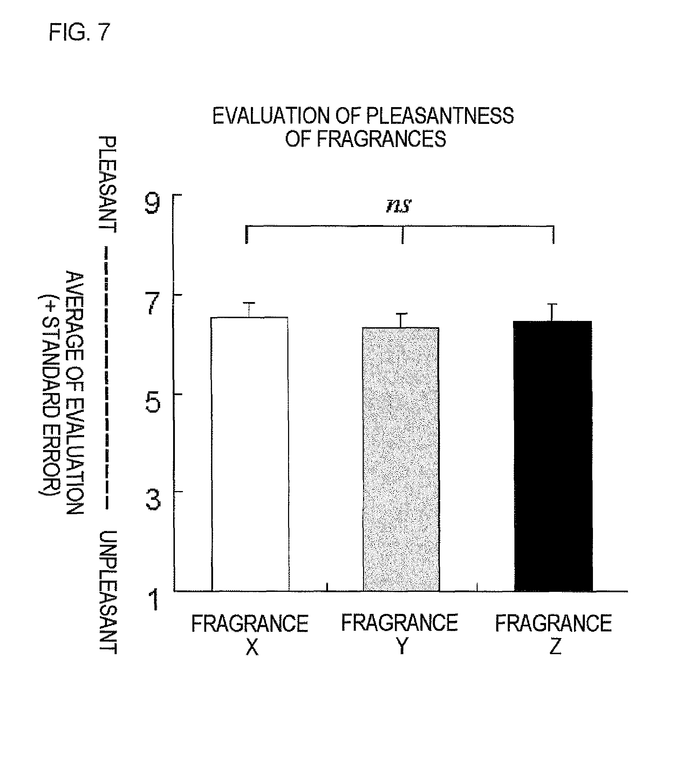

Three kinds of fragrances, listed in Table 4, were respectively applied to the body lotions having the same composition, and subjected to evaluation of preference by the subjects. The subjects were American women aged from 22 to 31, of which 43 smelled the body lotion applied with fragrance X (perfume X), 46 smelled the body lotion applied with fragrance Y (perfume Y), and 49 smelled the body lotion applied with fragrance Z (perfume Z). The preference was evaluated by asking the subjects to select a single item from a nine-grade criteria asking "How much do you like this fragrance?", ranging from "1: do not like very much" to "9: like very much".
The answer data regarding the preferences by the subjects were complied for each fragrance, and average values were calculated. FIG. 7 illustrates the average values of the scores of evaluation of pleasantness of the fragrances X to Z, evaluated in this process. The fragrance X to Z showed no significant difference.

**[Table 4]**

| Fragrance | Note |
|---|---|
| Fragrance X | Floral-powdery note |
| Fragrance Y | Floral-fruity note |
| Fragrance Z | Gourmand note |

### (Processes S2 to S4)

Next, the three kinds of candidates listed in Table 4 were respectively applied to the body lotions, the body lotions were presented to the subjects, and the subjects were allowed to smell them. All of the body lotions herein have the same composition. The subjects were American women aged from 22 to 31 who are the subjects in the evaluation of preference. Also the number of the subjects recruited for each fragrance was the same in the evaluation of preference.

Similarly to Example 1, the subjects were asked to smell the fragrance of the individual body lotions, allowed to recollect memory relevant to the subjects themselves in the past for each candidate, and to answer the degrees of recollection of memory. Also the items for evaluating the degree of recollection were the same as those in Example 1, which relate to the state of recollection (property of memory) and intensity thereof, and include three items with respect to the recollected memory induced upon smelling of the fragrances, such as, how much the emotional intensity was (emotional intensity of memory, evaluation item 1), how strongly did you feel your sense of being brought back to the time and place your memory arose (sense of being brought back, evaluation item 2), and how clear or how ambiguous was the memory (clearness of memory, evaluation item 3). Also in the evaluation of the degrees of recollection, the subjects were asked to answer by selecting a number from a nine-grade criteria ranging from 1 to 9, similarly as in Example 1.

### (Process S5)

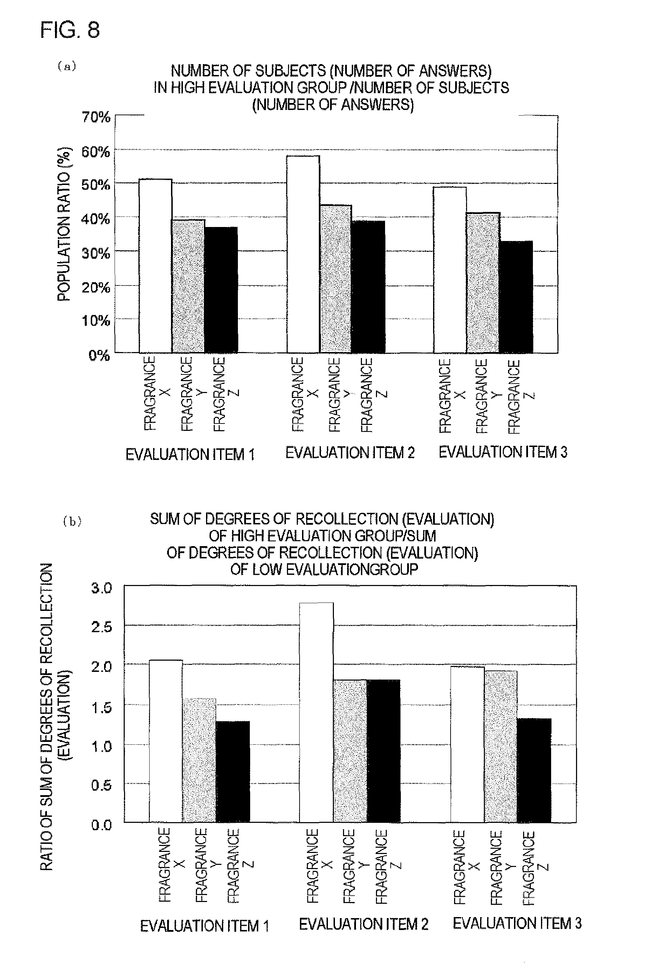

The answer data regarding the degrees of recollection given by the subjects were complied, and a fragrance to be applied to the target article was selected, similarly as in Example 1.
First, similarly as in Example 1, for each evaluation item, the answer data representing low evaluation from grade 1 to grade 5 were classified into the low evaluation group, and the answer data representing a high evaluation from grade 6 to grade 9 were classified into the high evaluation group.
Table 5 shows, for each of the evaluation items of the degrees of recollection of fragrances X to Z, similarly as in Example 1, the number of answers (the number of subjects) in the high evaluation group and population ratio thereof are relative to the number of all subjects (values in the parentheses in Table 5), and, the sum of the answer data of the high evaluation group, the ratio of the sum of the answer data of the high evaluation group relative to the sum of the answer data of the low evaluation group, and the ratio of the sum of the answer data of the high evaluation group relative to the sum of the answer data. On the other hand, FIG. 8(a) illustrates ratios of the number of answers by the high evaluation group relative to the number of all subjects, with respect to the evaluation items of the degree of recollection for the fragrances X to Z, and FIG. 8(b) illustrates ratios of sum of the answer data of the high evaluation group relative to the sum of the answer data of the low evaluation group (sum of the answer data of the high evaluation group/sum of the answer data of the low evaluation group), with respect to the evaluation items of the degree of recollection for the fragrances X to Z.

**[Table 5]**

| | Number of subjects | Evaluation item 1 | | | | Evaluation item 2 | | | | Evaluation item 3 | | | |
|---|---|---|---|---|---|---|---|---|---|---|---|---|---|
| | | High evaluation group | | | | High evaluation group | | | | High evaluation group | | | |
| | | Number of answers (population ratio) | Sum of degree of recollection (*1) | Ratio (*2) | Ratio (*3) | Number of answers (population ratio) | Sum of degree of recollection (*1) | Ratio (*2) | Ratio (*3) | Number of answers (population ratio) | Sum of degree of recollection (*1) | Ratio (*2) | Ratio (*3) |
| Fragrance X | 43 | 22(52%) | 159 | 2.06 | 67% | 25(58%) | 181 | 2.78 | 74% | 21(49%) | 150 | 1.97 | 66% |
| Fragrance Y | 46 | 18(39%) | 135 | 1.57 | 61% | 20(43%) | 148 | 1.80 | 64% | 19(41%) | 142 | 1.92 | 66% |
| Fragrance Z | 49 | 18(37%) | 126 | 1.27 | 56% | 19(39%) | 144 | 1.80 | 64% | 16(33%) | 116 | 1.32 | 57% |

| | | | | | | | | | | | | | |
|---|---|---|---|---|---|---|---|---|---|---|---|---|---|
| Item 1: Evaluation of emotional intensity of memory given by the subjects relevant to the subjects themselves in the past. Item 2: Evaluation of intensity of sense of being brought back to the time when memory given by the subjects relevant to the subjects themselves in the past arose. Item 3: Evaluation of clearness of memory given by the subjects relevant to the subjects themselves in the past. Number of answers: the number of answers by the high evaluation group (the number of subjects in the high evaluation group), Population ratio=(the number of answers by the high evaluation group)/(the number of all subjects) (*1): Sum of the degrees of recollection given by the subjects in the high evaluation group (*2): (Sum of the degrees of recollection given by the subjects in the high evaluation group)/(sum of the degrees of recollection given by the subjects in the low evaluation group) (*3): (Sum of the degrees of recollection given by the subjects in the high evaluation group)/(sum of the degrees of recollection given by the subjects) | | | | | | | | | | | | | |

As shown in Table 5, fragrance X was given a large number of subjects in the group representing higher degrees of recollection, with respect to the emotional intensity of memory given by the subjects relevant to the subjects themselves in the past (evaluation item 1), and also given a large value of {sum of the degrees of recollection (answer data) of the high evaluation group/sum of the degrees of recollection (answer data) of the low evaluation group}. The fragrance X was found to be given a larger population ratio of the high evaluation group, as compared with the other fragrances. The fragrance X was also given a larger value of the rate which is {sum of the degrees of recollection of the high evaluation group/sum of the degrees of recollection of the low evaluation group}, for all evaluation items, as compared with the other fragrances. As for {sum of the degrees of recollection of the high evaluation group/sum of the degrees of recollection given by all subjects}, fragrance X had higher scores as compared with the other fragrances for the evaluation items 1 and 2, but had a score equivalent to that of the fragrance Y for the evaluation item 3.
These results teach that the target article scented with the fragrance X was given a large number of subjects whose degrees of recollection of memory relevant to the subject themselves in the past are high.
While the Fragrances X to Z showed no distinctive difference as for the scores of evaluation of preference (pleasantness of fragrances) illustrated in FIG. 7, the fragrance X showed higher scores of evaluation with respect to the evaluation items regarding the memory given by the subjects relevant to the subjects themselves in the past, and this enabled the selection of the fragrance X which could not be selected based on the evaluation of preference.

### (Examination)

Next, performances of the target article were evaluated, similarly as in Example 1. The evaluation of the product performances of the target article was conducted by asking the subjects to answer any one value from grade 1 to grade 9, for each of 20 product performance evaluation items listed in Table 3, similarly as in Example 1

Again similarly as in Example 1, the data of the product performance evaluation obtained after use of the target article, were compiled together with answer data regarding the degrees of recollection of memory relevant to the subjects themselves in the past and recollected before use.

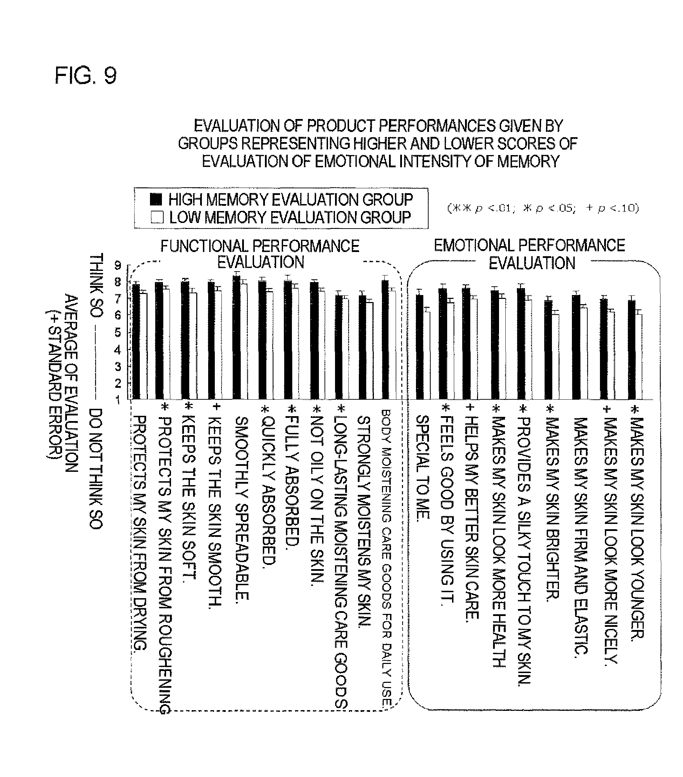

FIG. 9 illustrates the average values of the product performance evaluation given by the high evaluation group representing high scores of evaluation of the evaluation item 1, and the average values of the product performance evaluation given by the by the low evaluation group representing low scores of evaluation of the evaluation item 1, with respect to all of the fragrances X to Z. The evaluation item 1 is the emotional intensity of memory relevant to the subject themselves in the past and recollected before use. The evaluation item 1 is one of the evaluation items regarding the degrees of recollection of memory relevant to the subjects themselves in the past.
As illustrated in FIG. 9, the product performance evaluation given by the subjects in the group representing high degrees of recollection for the evaluation items 1, was found to be higher than the product performance evaluation given by the subjects in the group representing low degrees of recollection, and significant differences (p<0.05) were confirmed in five items in the emotional performance evaluation. It is considered that high product performance evaluation is obtained, when the degrees of recollection of memory relevant to the subjects themselves in the past and recollected before use, are high.

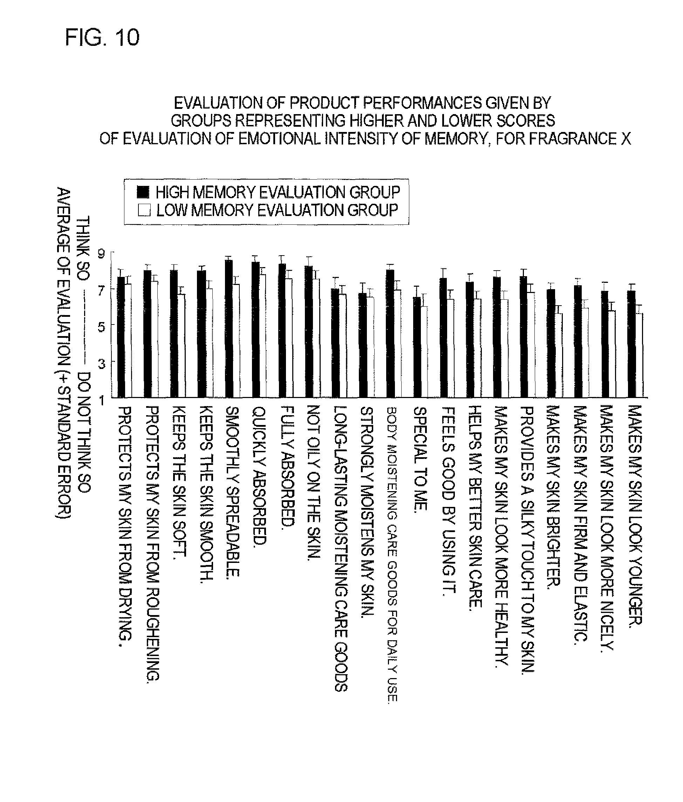

FIG. 10 illustrates average values of the product performance evaluation with respect to the fragrance X, given by the groups representing high and low degrees of recollection of memory relevant to the subjects themselves in the past (evaluation item 1). The group representing high degrees of recollection was found to provide high scores of evaluation in all items of product performance evaluation.

As is taught by the results of Example 1 and Example 2, the subjects whose degrees of recollection of memory are high give high scores of the product performance evaluation. According to the present invention, it is now enabled to select (a) fragrances, by which the product may highly be appreciated, by selecting any fragrances given large population ratios of the number of subjects in the group representing high degrees of recollection of memory, any fragrances given large values of {sum of the degrees of recollection (answer data) of the high evaluation group/sum of the degrees of recollection (answer data) of the low evaluation group}, and any fragrances given large values of {sum of the degrees of recollection of the high evaluation group/sum of the degrees of recollection given by all subjects}.

## Claims

1. A method of selecting a fragrance to be applied to a product, from a plurality of candidates, the method comprising the steps of:
presenting a plurality of products respectively scented with the plurality of candidates to a plurality of subjects;
asking the subjects to answer, with respect to the individual candidates, degrees of recollection of memory which are relevant to the subjects themselves in the past and recollected by smelling of the candidates, without presenting information regarding memory to be recollected;
classifying, with respect to each candidate, a plurality of degrees of recollection of memory obtained from the plurality of subjects into a group representing larger values of the degree of recollection of memory, and a group representing smaller values of the same; and
selecting a fragrance to be applied to the product, based on one, or two or more, scores of evaluation selected from (A), (B) and (C) below used as index(es):
(A) ratio of the number of all subjects, and the number of subjects in the group representing larger values of the degree of recollection of memory, for each candidate;
(B) ratio of the sum of the degrees of recollection given by the subjects in the group representing larger values of the degree of recollection, and the sum of the degrees of recollection given by the subjects in the group representing smaller values of the degree of recollection, for each candidate; and
(C) ratio of the sum of the degrees of recollection given by the subjects in the group representing larger values of the degree of recollection, and the sum of the degrees of recollection given by the all subjects, for each candidate.

2. The method of selecting a fragrance according to Claim 1,
wherein the degrees of recollection of memory relevant to the subjects themselves in the past are obtained by evaluating one or more items selected from intensity of emotion recollected by memory, intensity of sense of being brought back to the past, and clearness of memory.

3. The method of selecting a fragrance according to Claim 1 or 2,
wherein the step of presenting the plurality of products respectively scented with the plurality of candidates to the plurality of subjects, and the step of asking the subjects to answer degrees of recollection of memory, are conducted before use of the product by the subjects.

4. The method of selecting a fragrance according to any one of Claims 1 to 3,
wherein in the step of selecting a fragrance to be applied to the product, the classification into the group representing larger values of the degree of recollection of memory, and the group representing smaller values of the same, is based on either of the methods (I) and (II) below:
(I) using the median value of a numerical range of a multi-grade evaluation as a reference; and
(II) assuming the top 20 to 50% of the numerical range of the multi-grade evaluation as the group representing larger values, and the residual as the group representing smaller values.

5. The method of selecting a fragrance according to any one of Claims 1 to 4, further comprising:
selecting the plurality of candidates, which precedes the step of presenting the plurality of products respectively scented with the plurality of candidates to the plurality of subjects, the step of selection comprising:
a procedure of presenting a fragrance group, which contains a larger number of fragrances including the above-described plurality of candidates, to subjects;
a procedure of obtaining answers from the subjects about preference of each fragrance contained in the fragrance group; and,
a procedure of selecting a partial plurality of fragrances from the fragrance group, in decreasing order of preference including the highest preference, as the candidates.

6. The method of selecting a fragrance according to any one of Claims 1 to 5,
wherein, in the step of asking the subjects to answer the degrees of recollection of memory relevant to the subjects themselves in the past, a visible or audible question is directed to the subjects, in order to induce, upon smelling of the fragrance, memory relevant to the subjects themselves in the past.
